# EUROPÄISCHE PATENTSCHRIFT

(11) **EP 2 152 785 B1**
(45) Veröffentlichungstag und Bekanntmachung des Hinweises auf die Patenterteilung: **23.05.2012**
(21) Anmeldenummer: 08773291.3
(22) Anmeldetag: 03.06.2008
(51) Int. Cl.: A61L 27/58, D01F 6/76, A61L 27/00, C08G 77/02, C08L 83/02, C09D 183/02, C03B 37/00, C12N 11/14, A61L 27/18

(54) **NICHT-TOXISCHES POLYETHOXYSILOXAN-MATERIAL ZUR HERSTELLUNG VON BIOLOGISCH RESORBIERBARES UND/ODER BIOAKTIVES POLYETHOXYSILOXAN-MATERIAL ENTHALTENDEN ARTIKELN, DESSEN HERSTELLUNG UND VERWENDUNG**
NONTOXIC POLYETHOXYSILOXANE MATERIAL FOR THE PRODUCTION OF BIOLOGICALLY RESORBABLE AND/OR BIOACTIVE ARTICLES CONTAINING POLYETHOXYSILOXANE MATERIAL, THE PRODUCTION THEREOF, AND THE USE THEREOF
MATÉRIAU POLYÉTHOXYSILOXANE NON TOXIQUE POUR FABRIQUER DES ARTICLES CONTENANT UN MATÉRIAU POLYÉTHOXYSILOXANE BIOLOGIQUEMENT RÉSORBABLE ET/OU BIO-ACTIF, SA FABRICATION ET SON UTILISATION

(30) Priorität: 04.06.2007 DE 102007026043
(43) Veröffentlichungstag der Anmeldung: 17.02.2010
(73) Patentinhaber: Bayer Innovation GmbH, 40225 Düsseldorf (DE)
(72) Erfinder: Thierauf, Axel, 84066 Mallersdorf (DE)
(86) Internationale Anmeldenummer: PCT/DE2008/075002
(87) Internationale Veröffentlichungsnummer: WO 2008/148384

(56) Entgegenhaltungen:
- WO-A-2006/069567
- DE-C1- 19 609 551

## Beschreibung

Die Erfindung betrifft ein nicht-toxisches Polyethoxysiloxan-Material (PES-Material), ggf. ein gereiftes Polyethoxysiloxan-Material (rPES-Material), das bevorzugterweise als eines von mehreren verschiedenen Polyethoxysiloxan-Materialien (PES-Materialien) ausgebildet ist. Ein solches rPES-Material (r steht für reif, gereift) läßt sich erfindungsgemäß beispielsweise zu biologisch resorbierbaren und/oder bioaktiven Fasern als eines der PES-Materialien verspinnen und dann zu Vliesen als andere PES-Materialien weiter verarbeiten. Die vorliegende Erfindung betrifft weiterhin Verfahren zur Herstellung des ggf. gereiften PES-Materials, der biologisch resorbierbaren und/oder bioaktiven PES-Materialien und Verwendungen für diese Materialien.

Es gibt vielfältige Bestrebungen, biologisch resorbierbare Materialien für verschiedene Anwendungen in der Humanmedizin und der Medizintechnik, aber auch in anderen technischen Bereichen wie der Filtertechnik, der Biotechnologie oder der Dämmstoffindustrie zu entwickeln. In diesen Bereichen werden zudem stetig steigende Anforderungen, insbesondere an die biologische Aktivität und die toxikologischen Eigenschaften der Materialien, gestellt.

Resorbierbare Si-Polymere sind im Stand der Technik bekannt. In der DE 196 09 551 C1 sind biologisch degradier- und resorbierbare Faserstrukturen beschrieben. Diese Fasern können in einem Sol-Gel-Prozess dadurch erhalten werden, dass man aus einer Spinnmasse Fasern zieht und diese gegebenenfalls trocknet. Die Spinnmasse enthält eine oder mehrere partiell oder vollständig hydrolytisch kondensierte Verbindungen des Siliziums, die sich durch hydrolytische Kondensation ableiten von Monomeren der allgemeinen Formel SiX₄. Die Fasern haben den Nachteil, dass sie bei einer Degradation direkt nach dem Spinnprozess keine optimalen Ergebnisse in Cytotoxizitätstests zeigen und teilweise sogar als cytotoxisch eingestuft werden müssen. Eine solche Toxizität ist gerade im Einsatz in der Humanmedizin, in der Medizintechnik, in der Filtertechnik, in der Biotechnologie oder Dämmstoffindustrie, insbesondere im Bereich der Wundheilung oder der Filtration von Zellen aus Körperflüssigkeiten, gänzlich unerwünscht.

Das Verfahren zur Herstellung der Fasern nach der DE 196 09 551 C1 hat darüber hinaus den Nachteil, dass die resultierende Mischung nach dem Entfernen des Lösungsmittels im Hydrolyse-Kondensationsschritt ein mehrphasiges Gemisch ist und zum Entfernen des entstandenen Feststoffs einer Filtration unterworfen werden muss. Andere flüssige Si-Polymere, die ggf. toxisch sind, lassen sich mit einer Filtration überhaupt nicht entfernen. Darüber hinaus geht unter anderem durch die Bildung der festen Phase und durch den zwingenden Filtrationsschritt ein großer Anteil des spinnbaren Sols verloren. Nach dem Verfahren der DE 196 09 551 C1 kann auch während der Reifung die Bildung eines nicht unbeträchtlichen Anteils einer gelartigen Phase aus höherkondensierten Si-Verbindungen erfolgen. Dies reduziert nochmals den Anteil an spinnbarer Sol-Masse.

Aufgabe der vorliegenden Erfindung ist es, ein nicht-toxisches, biologisch resorbierbares und/oder bioaktives Material, dieses Material enthaltende Materialien und ein Verfahren zur Herstellung eines derartigen nicht-toxischen Materials zur Verfügung zu stellen.

Bioaktivität bedeutet erfindungsgemäß eine positive Interaktion zwischen Material bzw. Materialien einerseits und Gewebe (z.B. Wundgewebe) andererseits mit nachfolgender Differenzierung des Gewebes und als Folge davon Bindung oder Adhäsion von Gewebe entlang der Grenzfläche Material bzw. Materialien/(Empfänger-)Gewebe.

Die Aufgabe wird durch ein Sol bzw. eine Mikroemulsion (kolloidale Lösung) gemäß Anspruch 1 gelöst, die erfindungsgemäß auch als PES-Material bezeichnet wird. Eine derartige Mikroemulsion/kolloidale Lösung wird dadurch erhalten, dass
(a) eine erste Hydrolyse-Kondensationsreaktion (HKR) von höchstens einem Rest X von einer oder von mehreren verschiedenen Si-Verbindungen der Formel I

   SiX₄ (I),

   in der die Reste X gleich oder verschieden sind und Hydroxy, Wasserstoff oder Ethoxy (EtO) bedeuten, sauer katalysiert bei einem anfänglichen pH-Wert von 0 bis ≤ 7, in Gegenwart von Ethanol (EtOH) oder eines Ethanol-Wasser-Gemisches als Lösungsmittel, über einen Zeitraum von 1 bis 24 h bei einer Temperatur von 0°C bis 78°C (Siedepunkt des Ethanol) durchgeführt wird,
(b) eine zweite HKR des in Schritt (a) erhaltenen Materials bei gleichzeitigem Entfernen des Lösungsmittels durch sukzessives Eindampfen in einem geschlossenen, gasdiffusionsdichten Behälter (Rotationsverdampfer) bei einem definierten Druck bevorzugt ein leichter Unterdruck von ca. 500 mbar, bei einer Temperatur von bevorzugterweise 50-78°C, besonders bevorzugt bei etwa 70°C, bis zu einer drastischen Viskositätserhöhung auf ca. 1 Pa·s und bis zur Gewichtskonstanz und der Bildung eines Cyclotetrasiloxans der Molmasse von 4 x ca. 114 = ca. 456 g der allgemeinen Formel ((SiO(OH)_{0,75}(OEt)_{1,25} x 1/64 H₂O)₄, durchgeführt wird;
(c) dieses PES-Material in einem geschlossenen, vorzugsweise gasdiffusionsdichten Behälter zügig in wenig Minuten bis wenigen Stunden, vorzugsweise innerhalb einer halben Stunde abgekühlt wird, und
(d) das aus (c) erhaltene PES-Material durch eine dritte HKR in ein rPES Material überführt wird.

Es bleibt hervorzuheben, dass das erfindungsgemäße (nicht-toxische, biologisch resorbierbare und/oder bioaktive) PES- bzw. rPES-Material hergestellt werden kann, ohne dass das Herstellungsverfahren einen oder mehrere Filtrationsschritte umfassen würde oder gar müsste. Dies ist ein wesentlicher Unterschied zu dem Verfahren, das aus der DE 196 09 551 C1 bekannt ist.

Gegebenfalls schließt sich an Schritt (d) eine vierte HKR als einer der folgenden Schritte (e1) bis (e4) an, mittels derer aus dem in Schritt (d) erhaltenen rPES-Material eines der PES-Materialien wie Faser (e1), Pulver (e2), Monolith (e3) oder Beschichtung (e4) erzeugt werden kann. Dementsprechend beinhalten diese Schritte die folgenden Maßnahmen:
(e1) das rPES-Material zu biologisch resorbierbaren und/oder bioaktiven Fasern zu verspinnen;
(e2) das Material aus Schritt (d) dadurch zu einem Pulver zu verarbeiten, dass das erhaltene rPES-Material einer Trocknung, insbesondere einer Lyophilisation, unterworfen und das getrocknete PES-Material zu einem Pulver zerkleinert (gemahlen) wird;
(e3) das rPES-Material aus Schritt (d) in eine Form zu gießen und zu trocknen;
(e4) das rPES-Material aus Schritt (d) auf einen zu beschichtenden Körper aufzutragen oder diesen in das rPES-Material einzutauchen.

Es ist besonders bevorzugt, wenn das rPES-Material/die rPES-Materialien bei Anwendung einen pH-Wert von 5 bis 7, insbesondere von ≥ 6, aufweist bzw. aufweisen, damit es/sie eine akzeptable (physiologische) Verträglichkeit besitzt/en. Unter pH 5 ist das Material schon aufgrund des sauren Charakters unverträglich. Da in Schritt (b) bis zur Gewichtskonstanz, das heißt, bis kein oder fast kein Wasser mehr vorhanden ist, eingedampft wird, lässt sich die Säure-Stärke im wasserfreien System nicht als ein bestimmter pH-Wert definieren. Vielmehr sollte die optionale Abpufferung (also Zusatz eines geeigneten Puffers oder einer Lauge) oder Verringerung der Säure-Stärke (z.B. im Fall der Salpetersäure durch Austreiben/Verdampfen von NO₂) in (b) so erfolgen, dass das letztlich nach (e) erhaltene rPES-Material bzw. die daraus geformten PES-Materialien beim Wässern einen pH-Wert von 5 bis 7, insbesondere von ≥ 6, aufweist/aufweisen.

Um dies zu erreichen, ist es bevorzugt, die Säure-Stärke in Schritt (b) zu verringern bzw. die Wirkung der Säure abzupuffern. Erfolgt dies jedoch nicht bereits in Schritt (b), oder erfolgt dies nicht bis auf das bevorzugte Niveau, kann es auch nachfolgend in Schritt (c) oder (e) oder auch erst unmittelbar vor Applikation der PES-Materialien (z.B. auf der Haut bzw. Wunde) erfolgen. Die Einstellung der richtigen Säure-Stärke oder -Wirkung in Schritt (b) ist erfindungsgemäß aber eindeutig bevorzugt.

Die Verringerung der Säure-Wirkung in einem der Schritte (b), (c) oder (e) oder während der Wässerung der PES-Materialien kann insbesondere mittels Tris (Tris(hydroxymethyl)-aminomethan) in Form der freien Base oder eines Salzes (z.B. Tris-Acetat, Tris-Phosphat) erfolgen.

Nachfolgend werden die einzelnen Schritte der vorstehenden Reaktionen näher diskutiert.

### Schritt (a)

Erfindungsgemäß bevorzugt wird Tetraethoxysilan (TEOS) als einzige Si-Verbindung der Formel I in die erfindungsgemäße (erste) HKR eingesetzt.

Der anfängliche pH von 0 bis ≤ 7, vorzugsweise von 2-3,5, wird z.B mit verdünnter Salpetersäure (z.B. mit 1 N, vorzugsweise mit 0,01 N HNO₃) eingestellt. Geeignet sind aber grundsätzlich alle sauren Gemische und Lösungen, die geeignet sind, lokal NO oder NO₂ zu erzeugen. Dies können z.B. auch saure Gemische und Lösungen sein, die in physiologischer Umgebung mit molekularem Sauerstoff enzymatisch (mittels einer Nitroxid-Synthase, NOS) Stickstoffmonoxid (NO) erzeugen, das wiederum vom Körper schnell zu NO₂ umgesetzt wird oder es können auch Organische Nitrate bzw. Nitratester (so genannte NO-Donatoren) sein z.B. Ethylnitrat, die NO mit Hilfe einer organischen Nitratreduktase bilden. Für diese enzymatische Freisetzung von NO werden Thiolgruppen (Cystein) benötigt.

Neben der verdünnten Salpetersäure ist erfindungsgemäß deshalb auch eine wässrige oder alkoholische (besonders bevorzugt: eine wässrig verdünnte ethanolische) Lösung einer physiologisch verträglichen Säure (z.B. Zitronen-, Bernstein-, Wein-, Essig- oder Ascorbinsäure) und mindestens einer essentiellen Aminosäure (z.B. L-Arginin, besonders bevorzugt; L-Valin, L-Leucin, L-IsoLeucin, L-Phenylalanin, L-Thyroxin, L-Methionin, L-Lycin oder L-Tryptophan) oder einer nicht-essentiellen Aminosäure (z.B. L-Glutamin, L-Glutaminsäure, L-Asparagin, L-Asparaginsäure, L-Cystein, L-Glycin, L-Alanin, L-Prolin, L-Histidin, L-Tyrosin) als Substrat der NOS geeignet, den pH auf den gewünschten Wert im schwach bis mittelstark sauren Bereich einzustellen.

Wird zur Einstellung des pH verdünnte Salpetersäure (z.B. 0,01 N) verwendet, wird diese vorzugsweise eingesetzt in einem molaren Verhältnis Si-Verbindung(en) der Formel (I)/Salpetersäure von 110:1 1 bis 90:1, besonders bevorzugterweise von 100:1. Die Salpetersäure wird günstigerweise so eingesetzt, dass das molare Verhältnis Si-Verbindung der Formel I (z.B. TEOS)/HNO₃ etwa 100:1 1 beträgt.

Das Lösungsmittel, das in Schritt (a) erfindungsgemäß bevorzugt Verwendung findet, ist ein Ethanol-Wasser-Gemisch, das die Aufgabe hat, die Si-Verbindung(en) der Formel I zu lösen bzw. wenigstens zu emulgieren. Ist die Si-Verbindung der Formel I TEOS, löst/emulgiert Wasser die Si-Verbindung der Formel 1 nicht und wird deshalb vorzugsweise mit EtOH als Lösungsvermittler abgemischt. Die bevorzugte Menge an EtOH liegt bei 1 bis 1,5 Mol/Mol TEOS und beträgt gemäß einer besonders bevorzugten Ausführungsform 1,26 Mol/Mol TEOS.

Ein ganz besonders bevorzugter Reaktionsansatz wird erfindungsgemäß folgendermaßen durchgeführt. In dem Reaktionsgefäß wird 1 Mol TEOS vorgelegt, wozu dann 1,26 Mol EtOH gegeben werden. Dieses Gemisch wird gerührt, so dass der EtOH das TEOS löst. Separat werden 27,81 g 1 N HNO₃ (entspricht 1,75 g HNO₃) mit 60,38 g H₂O verdünnt (die Gesamtmasse der verd. Salpetersäure ist also 88,19 g, davon entfallen 86,44 g auf H₂O, entsprechend 4,8 Mol, und 1,75 g auf HNO₃, entsprechend 0,028 Mol; das Mol-Verhältnis H₂O/HNO₃ beträgt 4,8/0,028 = 172). Von der verdünnten Salpetersäure werden dann 33,07 g zu der ethanolischen TEOS-Lösung gegeben (so dass 1,8 Mol H₂O und 0,01 Mol HNO₃ pro 1 Mol TEOS eingesetzt werden).

Die erste HKR läuft exotherm ab. Die erste HKR bedeutet erfindungsgemäß, erläutert am Beispiel von TEOS, das je eine EtO-Gruppe in je einem Molekül TEOS hydrolysiert und die resultierende OH-Gruppe unter Dimerisierung und Wasser-Abspaltung unter ständigem Rühren kondensiert. Das heißt, man vereinigt die beiden Lösungen (z.B. TEOS in EtOH und verd. Salpetersäure) bei Raumtemperatur (RT), wobei die Temperatur während der Umsetzung von 2 SiX₄ (also z.B. von 2 TEOS) via Hydrolyse und Kondensation von je einem EtO-Rest zu X₃Si-O-SiX₃ (z.B. (EtO)₃-Si-O-Si-(EtO)₃) auf etwa 50-60°C ansteigt. Auf die anfängliche Temperatur bei der ersten HKR kommt es dabei nicht wesentlich an (da die Reaktion ohnehin exotherm abläuft). Sie kann RT sein, sie kann aber auch unterhalb oder oberhalb der jeweiligen RT , z.B. bei 5, 10, 15, 20, 35, 45, 60 oder 70°C liegen. Sie muss lediglich so hoch liegen, dass die erste HKR ablaufen kann.

Erfindungsgemäß besonders bevorzugt wird die Hydrolyse von mehr als einer EtO-Gruppe pro TEOS-Molekül vermieden. RT (etwa 20°C, ggf. 18-25°C) ist daher aus ökonomischen und praktischen Gründen bevorzugt. Höhere Temperaturen bis 78°C sind ebenfalls geeignet, solange sie im Bereich von 0°C bis 78°C, vorzugsweise im Bereich von 10°C bis 70°C oder im Bereich von 20°C bis 60°C liegen. Es gilt für die Temperatur natürlich die übliche Beziehung in der Chemie, dass eine tiefere Temperatur längere Reaktionszeiten - und umgekehrt - erfordert. In einer bevorzugten Ausführungsform der vorliegenden Erfindung wird diese erste HKR über einen Zeitraum von 1-12 Stunden durchgeführt. Besonders bevorzugt sind Zeiträume von 5-8 h.

Das Sol kühlt während des Rührens auf RT ab. Wenn das Sol RT erreicht hat und das Rühren beendet wurde, muss sich Schritt (b) anschließen. Das Reaktionsgemisch soll also nicht unnötig bei RT ohne Rühren herumstehen, ohne dass (b) durchgeführt wird. Andernfalls läuft die HKR weiter und das Gemisch bildet gel-artige, höher kondensierte Si-Verbindungen.

Die erste HKR wird vorzugsweise diskontinuierlich in einem Rührwerksbehälter durchgeführt. Die Si-Verbindung der Formel I (z.B TEOS) und das Solvens (z.B. Ethanol) werden vorzugsweise vorgelegt. Anschließend erfolgt die zügige Zugabe der Säure, vorzugsweise in Form von 0,01 N HNO₃ (z.B. 0,01 Mol HNO₃ pro Mol TEOS)). Aufgrund der Säure-Stärke in dem Reaktionsgemisch läuft die erste HKR schnell ab, und der Inhalt des Behälters erwärmt sich auf 50°C bis 60°C, ehe die Temperatur noch während der Reaktionszeit (also in Schritt (a)) zu sinken beginnt (in Folge einer natürlichen Abkühlung auf die Umgebungstemperatur, d.h., ohne externe Kühlung).

### Schritt (b)

Durch eine zweite HKR des in Schritt (a) erhaltenen Materials in einem geschlossenen, gasdiffusionsdichten Behälter (Rotationsverdampfer) bei gleichzeitiger Entfernung des Lösungsmittels (Wasser, Ethanol) durch sukzessives Eindampfen bei einem Druck von 100 bis 1013 mbar, bevorzugterweise bei 500 bis 800 mbar, einer Reaktionstemperatur von RT bis 70°C, vorzugsweise 60-70°C und bevorzugterweise unter langsamem Rotieren von 20 U/min bildet sich unter drastischer Erhöhung der Viskosität Cyclotetrasiloxan der allgemeinen Formel: ((SiO(OH)_{0,75}(OEt)_{1,25} x 1/64 H₂O)₄ mit einer Molmasse von 4 x ca 114 g = ca. 456 g aus.

Schritt (b) sollte unbedingt unter Wasserausschluss ablaufen, damit keine weitere Hydrolyse erfolgen kann. Wasserausschluß in diesem Kontext bedeutet hier, daß kein weiteres Wasser von außen zugesetzt wird oder via Luftfeuchte in das Reaktionsgemisch gelangt; auf Grund der Kondensationsreaktion, bei der Wasser gebildet wird, und des ggf. als Lösungsmittel in Schritt (a) zugesetzten Wassers ist das Reaktionsgemisch in Schritt (b) jedoch nicht wasserfrei.

Die Eindampftemperatur sollte 78°C (Siedepunkt EtOH) nicht überschreiten, da das Lösungsmittel(gemisch) ansonsten Blasen bildet. Temperaturen oberhalb von 60°C sind besonders bevorzugt, da bei tieferen Temperaturen die Säure, im Falle von HNO₃ das NO₂, nicht mehr in ausreichendem Masse abgedampft werden kann und die weitere HKR wesentlich saurer verlaufen würde und letztlich eine höhere Säure-Konzentration im Material verbleiben würde.

Entsprechend dieser Erkenntnis der Erfinder ist es besonders bevorzugt, Schritt (b), den Schritt des sog. Reaktiveindampfens, so lange ablaufen zu lassen, bis die Viskosität sprunghaft auf ca. 1 Pa·s angestiegen ist und Gewichtskonstanz erreicht worden ist, hilfsweise solange bis ein Cyclotetrasiloxan vorliegt, vorzugsweise unter gleichzeitiger, möglichst weitgehender Verringerung der Säure-Stärke (durch Abdampfen der Salpetersäure bzw. des NO₂). Der Fachmann erkennt das Ende von Schritt (b) an der Viskosität, die stark anzusteigen beginnt und Werte von 0,5 - 2 Pa·s erreicht.

Bevorzugterweise wird (b) durch Abkühlen auf Temperaturen unterhalb 10°C, siehe unter Schritt (c)) beendet, wenn die Viskosität des Reaktionsgemisches bei etwa 1 Pa·s liegt. Es liegt dann eine einphasige, "warme" Mikroemulsion bzw. ein einphasiges, "warmes" Sol mit einer Viskosität von 0,5 bis 2 Pa·s bei einer Scherrate von 10 s⁻¹ bei 4°C (PES-Material) vor.

Das Reaktiveindampfen erfolgt bei Temperaturen, die die Entfernung von Wasser, EtOH und gegebenenfalls bei Verwendung von Salpetersäure, NO₂ erlauben. 70°C ist aus praktischer Sicht bevorzugt, aber auch leicht darunter liegende Temperaturen wie 50°C oder 60°C sind bevorzugt. Ohne dass sich die Erfinder an ihre theoretische Überlegung, gestützt durch erste experimentelle Daten, gebunden fühlen, postulieren sie für das in Schritt (b) erhaltene PES-Material ("warme" Mikroemulsion bzw. "warmes" Sol) eine Zusammensetzung von ((SiO(OH)_{0,75}(OC₂H₅)_{1,25} x 1/64 H₂O)₄ (MG = 4 x 113,28 g = 453,12 g) in Form von Viererformationen, die vier Si-O-Einheiten sind, also Achtringe darstellen.

Der aufmerksame Leser hat längst erkannt, dass während Schritt (b) die in (a) erhaltenen Intermediate unter Abspaltung von EtOH bzw. Wasser und deren nachfolgender Entfernung weiter reagieren. Wird Schritt (b) nicht im geschlossenen Gefäß oder nicht bis zur Gewichtskonstanz oder nicht bis zur Viskositätserhöhung (vorzugsweise auf 1 Pa·s) durchgeführt, bleiben physiologisch unerwünschte Substanzen in dem PES-Material zurück, die sich während der Schritte (c), (d) und/oder (e1)-(e4) nicht oder nicht ohne weiteres entfernen lassen.

Wird in Schritt (a) verdünnte Salpetersäure als Säure verwendet, erfolgt die mögliche und bevorzugte Verringerung der Säure-Stärke in Schritt (b) dadurch, dass sich die Säure während des Reaktiveindampfens zu NO₂, _{O2} und Wasser zersetzt. NO₂ (Sdp ~ 21,2°C) wird dann aber nur zum (allergrößten) Teil ausgetrieben, ein (sehr geringer) Teil verbleibt eingeschlossen in der Mikroemulsion/dem Sol. Wird aber das System organische Säure/Arginin an Stelle von Salpetersäure verwendet, erfolgt die Erhöhung des pH bzw. die Verringerung der Säure-Stärke, falls erwünscht, z.B. mittels Tris-Lösungen (soweit sich die Säure, z.B. Essigsäure, nicht austreiben läßt).

Es wurde nun überraschenderweise festgestellt, dass bei Einhaltung der Bedingungen, wie sie vorstehend für die Schritte (a) und (b) beschrieben worden sind, und nach dem Entfernen des Lösungsmittels in Schritt (b) eine Mikroemulsion erhalten wird, die vor der Reifung in Schritt (d) keiner Filtration mehr bedarf, d.h. einphasig ist.

### Schritt (c)

Dieser Schritt, der ein Abkühlvorgang ist, wird sinnvollerweise dadurch gekennzeichnet, dass die in Schritt (b) erhaltene "warme" Mikroemulsion zügig d.h. innerhalb weniger Minuten bis wenigen Stunden, vorzugsweise innerhalb einer halben Stunde, in einen geschlossenen, vorzugsweise in einen gasdiffusionsdichten Behälter überführt und auf diejenige Temperatur abgekühlt wird, bei der Schritt (d) durchgeführt wird.

Dementsprechend liegen die Temperaturen, auf die bevorzugt abgekühlt wird, bei -20°C bis 10°C, bevorzugt bei 2°C bis 4°C, besonders bevorzugt bei 4°C. Der Eintritt von Feuchtigkeit, z.B. als Luftfeuchtigkeit oder am Behälter anhaftende Feuchtigkeit, ist unbedingt zu vermeiden. Gegebenenfalls erfolgt in diesem Schritt auch die Anpassung des Materials derart, dass der pH-Wert der am Körper anzuwendenden späteren Materialien bei pH 5 bis 7 liegt, bevorzugt ist pH > 6.

### Schritt (d)

Die kinetische kontrollierte Reifung ist Bestandteil des erfindungsgemäßen Verfahrens und ermöglicht überhaupt erst die Verarbeitbarkeit in Form z.B. der Spinnbarkeit oder auch der Beschichtbarkeit des nach Schritt (c) erhaltenen Reaktionsgemisches (PES-Materials). In diesem Schritt (d) erfolgt, eine dritte HKR, wobei sich die Viskosität des Reaktionsgemisches dadurch erhöht, das sich die in Schritt (b) erzeugten Viererformationen (Cyclotetrasiloxane) zu Würfeln bzw. Käfigen (Silsesquioxane) der allgemeinen Formel Si₈O₁₂(OH)₂(EtO)₆ mit acht Si-Atomen an den Ecken und zwölf Sauerstoff-Brücken an den Kanten zusammenlagern. In Abhängigkeit von der zu erzielenden Viskosität, bilden sich also aus den Silsesquioxanen, Ketten aus Würfeln/Käfigen bzw. Oligosilsesquioxane.

Schritt (d) erfolgt erfindungsgemäß in geschlossenen vorzugsweise gasdiffusionsdichten Gefäßen, z.B. in so genannten Reifebechern, vorzugsweise in den Gefäßen, die bereits für Schritt (c) verwendet wurden. Der Eintritt von Feuchtigkeit oder anderen Gasen, hier sei auch CO₂ erwähnt, ist unbedingt zu vermeiden. Die bevorzugte Durchführung von Schritt (d) erfolgt erfindungsgemäß bei einer Temperatur von (oberhalb) -20°C bis 10°C in einem Zeitraum von 1 Tag bis zu 4 Wochen, bevorzugt bei 2°C bis 4°C und in einem Zeitraum von 3 bis 18 Tagen. Besonders bevorzugt wird die Reifung über einen Zeitraum von 3 bis 5 Tagen bei 4°C durchgeführt, insbesondere durch erschütterungsfreies Lagern des Reaktionsgemisches in geschlossenen, vorzugsweise gasdiffusionsdichten Gefäßen. Die Reifung kann aber genauso bevorzugt bei jeder Temperatur im Bereich von (oberhalb) -20°C bis 10°C erfolgen.

Der Fachmann erkennt, dass Temperatur und Reaktionszeit zwei voneinander abhängige Größen sind, die aufeinander abgestimmt werden, vorzugsweise so, dass das in Schritt (c) erhaltene Material sich vollständig zu einem Silsesquioxan der allgemeinen Formel Si₈O₁₂(OH)₂(EtO)₆ umgesetzt hat und das so in (d) erhaltene rPES-Material eine dynamische Viskosität annimmt, die es für die Durchführung eines der Schritte (e1) bis (e4) geeignet macht und vorbereitet. Soll das Material in einem Schritt (e1) zur Faser versponnen werden, sollte die dynamische Viskosität am Ende von (d) etwa 30 bis 55 Pa·s (Scherrate 10 s⁻¹ bei 4°C) mit einem Verlustfaktor von 3,5 betragen (der Verlustfaktor ist der Quotient aus elastischem und inelastischem Anteil der dynamischen Viskosität). Soll das Material dagegen in einem Schritt (e2) zum Pulver verarbeitet werden, beträgt die dynamische Viskosität am Ende von (d) etwa 60 Pa·s (Scherrate 10 s⁻¹ bei 4°C). Im Fall der Verarbeitung des Materials zu einem Monolith (in einem Schritt (e3)), beträgt die dynamische Viskosität am Ende von (d) vorzugsweise größer gleich 70 Pa·s (Scherrate 10 s⁻¹ bei 4°C). Und wenn das Material in einem Schritt (e4) zur Beschichtung von Körpern oder Oberflächen verwendet werden soll, liegt die dynamische Viskosität je nach gewünschter Schichtdicke bei kleiner gleich 10 Pa·s (Scherrate 10 s⁻¹ bei 4°C).

Durch die niedrige Temperatur während der Reifung im Reifebecher läuft, ausgehend von der Viererformation (Cyclotetrasiloxan), eine kinetisch kontrollierte Hydrolyse und Kondensation (die dritte HKR) ab, so dass sich Silsesquioxane der allgemeinen Form: Si₈O₁₂(OH)₂(EtO)₆ bilden Diese Silsesquioxane aggregieren über Wasserstoftbrücken-Bindungen.

Ohne dass sich die Erfinder an ihre theoretische Überlegung, gestützt durch erste experimentelle Daten, gebunden fühlen, postulieren sie für das in Schritt (d) erhaltene rPES-Material eine Zusammensetzung der folgenden Art: Während der Aggregation läuft die dritte HKR weiter ab, so dass sich die Silsesquioxane (Würfel) zu Oligosilsesquioxanen (Würfelketten) der allgemeinen Formel [OSi₈O₁₂(OH)₂(EtO)₅] zusammenlagern und zu einer bevorzugterweise ein-dimensionalen Ketten-Bildung führen. Diese Würfelketten bilden lineare (quasi-eindimensionale) oligomere Strukturen, die leicht 100-1000 nm Länge erreichen können. Die Würfelketten untereinander sind jeweils über Wasserstoffbrücken aggregiert und enthalten noch restliche Ethoxy-Gruppen. Makroskopisch drückt sich die ein-dimensionale Kettenstruktur in einer besonderen Form der Viskosität, der so genannten Strukturviskosität aus. Durch zunehmende HKR der Würfel zu Würfelketten erhöht sich die Viskosität weiter. Die Bildung der Würfelketten wird hierbei solange durchgeführt bis die gewünschte Viskosität vorliegt.

Das Endprodukt der Reifung im Reifebecher ist demgemäß ein unbegrenzt haltbares Sol (das rPES-Material) mit einer bestimmten Strukturviskosität. Strukturviskosität ist die Eigenschaft eines Fluids, bei hohen Scherkräften eine niedrigere Viskosität zu zeigen; je stärker die Scherung ist, die auf das Fluid wirkt, desto weniger viskos (zähflüssig) ist es. Die Abnahme der Viskosität entsteht durch die Krafteinwirkung auf die Oligomere im Sol, die dafür sorgt, dass die einzelnen Sol-Partikel (hier Oligosilsequioxane) sich ausrichten und deshalb besser aneinander vorbeigleiten können; weiteres hierzu, insbesondere zur Größe und Form der Strukturen, die die Spinnfähigkeit ausmachen, siehe Sakka in Sol-Gel Technology for Thin Films, Fibers, Preforms, Electronics and Specialty Shapes, ed. L. C. Klein, Ncyes, Park Ridge, N. Y., 1988, Seite 140 und Abbildung 2.7.).

Vorteilhafterweise wird erfindungsgemäß also die konkurrierende Ausbildung eines dreidimensionalen polymeren Gel-Netzwerkes (weitestgehend) unterdrückt, das Endprodukt des erfindungsgemäßen Verfahrens nach Schritt (d) ist vorteilhafterweise also ein hydrophobes, Ethoxy-Gruppen aufweisendes einphasiges Sol aus Oligosilsesquioxanen (Würfelketten) ohne Gel-Anteil, das (weitestgehend) frei von Wasser ist und sich bestens für die dauerhafte Lagerung, Transport und Vertrieb eignet.

Da die kinetisch kontrollierte Reifung, also Schritt (d), unterhalb von -20°C gar nicht oder allenfalls minimal abläuft, kann das PES-Material nach Schritt (c) bei -20°C "eingefroren" werden (es ist bei dieser Temperatur ebenfalls unbegrenzt haltbar). Das ist insofern durchaus eine bevorzugte Variante, als sich das PES-Material (vor Schritt (d)) so genauso lagern und transportieren lässt wie das rPES-Material nach Schritt (d).

Wiederum ohne dass sich die Erfinder an theoretische Überlegungen und vorläufige experimentelle Daten gebunden fühlen, postulieren sie für das in Schritt (d) erhaltene rPES-Material ein Silsesquioxan der allgemeinen Formel Si₈O₁₂(OH)₂(EtO)₆ bzw. ein Oligosilsesquioxan mit der Zusammensetzung [OSi₈O₁₂(OH)₂(EtO)₅].

Silsesquioxane erhalten ihren Namen von dem 1,5-zählige oder sesqui-stöchiometrischen Sauerstoff pro Silicium-Atom. Für diesen Verbindungstyp ist eine Reihe von geometrischen Strukturen bekannt, unter anderem Leiter, Kubus oder Käfigstrukturen. Ein voll kondensiertes Silsesquioxan hat die Struktur [RSiO_{1,5}]ₙ und wird polyedrisches oligomeres Silsesquioxan (polyhedral oligomeric silsesquioxan. POSS) genannt. Völlig durchkondensierte POSS sind literaturbekannt und kommerziell erhältlich (z.B. bei Sigma-Aldrich, St. Louis, MO, USA) mit einer großen Zahl an Substituenten. Sigma-Aldrich bietet z.B. ein Octamethyl-POSS der Formel (CH₃)₈Si₈O₁₂ sowie ein Cyclopentyl-POSS-Silanol der Formel (C₅H₉)₇Si₈O₁₂(OH) an, die beide hinlänglich verschieden sind von dem erfindungsgemäßen Oligosilsesquioxan [OSi₈O₁₂(OH)₂(EtO)₅]. Ein derartiges Pentaethoxy-POSS-Silanol ist auch in der Literatur nicht bekannt (für einen guten Überblick siehe http://www.sigmaaldrich.com/aldrich/brochure/al_chemfile_v1_no6.pdf).

Bekannt sind auch Silsesquioxane der empirischen Formel RSiO_{1,5}, wobei die Substituenten R theoretisch folgende Gruppen sein können: Wasserstoff, Hydroxyl, Alkyl-, Alkenyl-, Alkoxy-, and Aryl-Reste. Literaturbekannt sind unter anderem Silsesquioxane mit folgenden Substituenten R: Methyl, Propyl, Allyl, Methacryl, Phenyl, Wasserstoff, Hydroxyl.

### Schritt (e1)

Der Spinnprozess zur Verarbeitung des Sols zur Faser wird unter üblichen Bedingungen durchgeführt, wie zum Beispiel in DE 196 09 551 C1 und DE 10 2004 063 599 A1 beschrieben. Die dynamische Viskosität des Sols liegt vorzugsweise bei 30 bis 55 Pa·s (Scherrate 10 s⁻¹ bei 4°C), der Verlustfaktor bei 3,5. Bei dem Spinnprozess wird das rPES über einen Druckbehälter durch einen Düsenkopf mit bis zu 100 Einzeldüsen ausgeblasen (Druck im Behälter 1-100 bar, vorteilhafterweise 20 bar). Die letztlich resultierende Faser besteht in der Regel aus Würfelketten (Oligosilsesquioxanen) der allgemeinen Formel [OSi₈O₁₂(OH)₂(EtO)₅], die während des Spinnvorgangs quer miteinander vernetzen. Das aus der (kalten) Düse austretende Sol erfährt beim Durchfallen durch den (warmen) Spinnschacht eine weitere (vierte) HKR, die dafür verantwortlich ist, dass der aus der Düse austretende Strahl via (molekularer) Quervernetzung der Oligosilsesquioxane zu einer (stabilen) Faser reagiert. Der Spinnschacht hat üblicherweise eine Länge von 1-5 m, vorteilhafterweise 2 m. Das Klima im Spinnschacht wird bzgl. Temperatur und Feuchtigkeit kontrolliert eingestellt, ggf. kann hier auch eine Atmosphäreneinstellung (etwa 20°C und etwa 35 % (33-37 %) Luftfeuchte) mit weiteren Reaktanden (z.B. Ethylnitrat) erfolgen

Die Fasern sind nach dem Durchfallen durch den Spinnschacht im Querschnitt rund (nicht oval oder gar knochenförmig), haben kein Wellenprofil im Längsschnitt und sind formstabil. Sie werden auf einen Changiertisch abgelegt. Die Maschenweite der so entstehenden Fasergelege wird über die Changiergeschwindigkeit eingestellt. Diese liegt bei einigen cm/min. Durch langsamen Vortrieb entsteht so ein engmaschigen Fasergelege (Vlies), bei dem, bezogen auf TEOS als Si enthaltende Ausgangsverbindung, noch über 30 % der Ethoxy-Gruppen vorhanden sind..

Die in Schritt (e1) erfindungsgemäß hergestellten Fasern weisen aufgrund der noch vorhandenen Ethoxy-Gruppen eine gewisse Hydrophobizität auf. Sie sind darüber hinaus (weitestgehend) frei von Lösungsmittel (Wasser, Ethanol) und eignen sich bestens für die dauerhafte Lagerung, den Transport und Vertrieb. In der Tat besteht eine bevorzugte Ausführungsform der Erfindung darin, die Fasern bzw. Vliese nach Schritt (e1) bzw. das Pulver, den Monolith und die beschichteten Körper/Oberflächen nach Schritt (e2), (e3) und (e4) herzustellen und diese erfindungsgemäßen Ausführungsformen zu lagern, zu transportieren und zu vertreiben.

Wird in Schritt (a) verdünnte Salpetersäure als Säure verwendet, erfolgt die mögliche und bevorzugte Verringerung der Säure-Stärke in Schritt (e1), (e2), (e3) und (e4) dadurch, dass der verbliebene, eingeschlossene Teil der NO₂ (Sdp ~ 21,2°C) dann durch Ablüften bei bevorzugterweise 30°C entfernt wird. Wird aber das System organische Säure/Arginin an Stelle von Salpetersäure verwendet, erfolgt die Erhöhung des pH bzw. die Verringerung der Säure-Stärke, falls erwünscht, z.B. mittels Tris-Lösungen (soweit sich die Säure, z.B. Essigsäure, nicht austreiben läßt) kurz vor der Applikation durch spülen in einer wässrigen Tris-Lösung.

Es liegt auf der Hand, dass im Fall von (e4) Lagerung und Transport bevorzugterweise im "eingefrorenen" Zustand des PES-Materials nach Schritt (c) erfolgen.

### Schritt (e2)

Vor oder auch während der Trocknung kann das rPES-Material mit einer dynamischen Viskosität von etwa 60 Pa·s (Scherrate 10 s⁻¹ bei 4°C), erhalten aus Schritt (d) (das auf Grund seiner Bioaktivität als ein Wirkstoff betrachtet werden kann), mit beliebigen (weiteren) Wirkstoffen, beispielsweise pharmazeutisch wirksamen Substanzen versetzt oder mit einer weiteren, vierten HKR kovalent gebunden werden (nachfolgend ist bei Verwendung des Begriffes "Wirkstoff' jedoch regelmäßig nicht das rPES-Material aus Schritt (d) gemeint, sondern der weitere Wirkstoff). Vorzugsweise soll das unter Erzeugung eines homogenen Gemisches erfolgen. Insbesondere im Fall der Beimischung von temperaturempfindlichen Wirkstoffen wird das Gemisch aus PES-Material und Wirkstoff(en) nach der vierten HKR einer schonenden Trocknung, z.B. einer Sprüh- oder Gefriertrocknung unterworfen. Ist der Wirkstoff nicht temperaturempfindlich oder wird ein solcher gar nicht zugesetzt, kann die Trocknung auch bei (deutlich) erhöhten Temperaturen bewirkt werden. Dabei bildet sich vorzugsweise eine bioresorbierbare und/oder bioaktive Matrix um den Wirkstoff. Diese Matrix ist insbesondere auch für die Einkapselung von flüssigen Wirkstoffen geeignet (Flüssigkeiten können langzeitstabil in der Matrix eingeschlossen und kontrolliert wieder freigegeben werden). Die Einkapselung ermöglicht die mechanische und chemische Stabilisierung der Wirkstoffe, die verbesserte Handhabbarkeit solcher flüssigen Wirkstoffe und Arzneimittel und hilft, eine ungesteuerte Verflüchtigung der Wirkstoffe zu vermeiden. Es können selbstverständlich weitere, der jeweiligen Anwendung angepasste Substanzen und/oder Hilfsstoffe in der endgültigen Formulierung (Pulver) vorhanden sein. Anwendungen ohne zusätzlichen Wirkstoff sind z.B. Zuschlagstoffe für Hautcremes etc., wie sie z.B. in http://www.photolagen.com/ beschrieben sind.

Das Pulver kann ein Mikropulver und/oder ein Nanopulver sein. Die Partikel eines erfindungsgemäßen Mikropulvers haben, vorzugsweise eine Größe (einen mittleren Durchmesser) von 0,01 µm bis 100 µm, insbesondere 0,1 bis 20 µm. Die Nanopulver-Partikel haben in der Regel eine Größe (einen mittleren Durchmesser) von ≤ 100 nm.

### Schritt (e3)

In einer weiteren Ausgestaltung kann das rPES-Material (dynamische Viskosität am Ende von (d) vorzugsweise größer gleich 70 Pa·s bei einer Scherrate von 10 s⁻¹ bei 4°C) aus Schritt (d) (wiederum vor oder während der Trocknung) mit einem (weiteren) Wirkstoff, beispielsweise einer pharmazeutisch wirksamen Substanz versetzt oder mittels einer vierten HKR kovalent gebunden werden. Anschließend erfolgt dann, unabhängig von der Gegenwart des (weiteren) Wirkstoffs, das Gießen des rPES-Materials in eine Form. Nach der Trocknung kann auf diese Weise ein Monolith erhalten werden. Solche Monolithe können in Form von massiven Implantaten als Wirkstoff-Zulieferungs-System (Drug Delivery System) beispielsweise subkutan eingesetzt werden. Sie können beispielsweise als Depot für Contraceptiva eingesetzt werden und über einen längeren Zeitraum den Wirkstoff freigeben. Solche erfindungsgemäßen Implantate weisen eine gute biologische Verträglichkeit auf. Die Monolithe können vorzugsweise einen Durchmesser von ≥ 0,5 mm aufweisen. Alternativ können die Monolithe auch zu Pulver zerkleinert und gemahlen werden.

### Schritt (e4)

Das gereifte Material aus Schritt (d) kann aber auch zu einer Beschichtung verarbeitet werden. Dazu wird der zu beschichtende Körper durch Tauchen in das rPES-Material (dynamische Viskosität bei kleiner gleich 10 Pa·s; Scherrate 10 s⁻¹ bei 4°C), durch Begießen mit dem rPES-Material oder durch Aufschleudern oder Sprayen des rPES-Materials beschichtet. Bevorzugt als Beschichtungen sind solche auf Dragees oder Kapseln, wozu gepresste pulverförmige Arzneimittel-Gemische mit einer biologisch resorbierbaren und/oder bioaktiven Beschichtung aus dem rPES-Material versehen werden. Hierdurch kann die Freisetzung von (weiteren) Wirkstoffen (z.B. über die Schichtdicke und/oder die Schichtabfolge) innerhalb der Formulierung kontrolliert und/oder gesteuert werden. Eine derartige Beschichtung läßt sich aber auch auf Körperteil-Implantate (z.B. aus Titan) aufbringen, wodurch die (biologische) Verträglichkeit der Implantate verbessert wird, z.B. Abstoßungsreaktionen abgemildert oder verhindert werden.

Nach einer weiteren Ausgestaltung der Erfindung können hochviskose Sole, insbesondere Hydrogele, durch das erfindungsgemäße rPES-Material ergänzt oder ersetzt werden. Die hochviskosen Sole und die Hydrogele werden in der Medizin und in der Kosmetik als Wirkstoff- bzw. Arzneimittelträger verwendet. Generell werden Hydrogele vielfach in der Versorgung von großflächigen Wunden (Wundbehandlung und Wundheilung) eingesetzt. Vorteilhafterweise kann durch den Zusatz des rPES-Materials die biologische Verträglichkeit und damit die Wundheilung verbessert werden. Die erfindungsgemäßen Hydrogele können insoweit vorteilhaft als biologisch resorbierbare und/oder bioaktive Produkte in der Medizin, insbesondere Humanmedizin oder Medizintechnik eingesetzt werden.

### Weiterverarbeitung bzw. -Verwendung der Faser

Die Fasern als Endprodukte eines der erfindungsgemäß bevorzugten Verfahren (umfassend die Schritte (a) bis (d), und (e1)) können als Fasern aber auch als Vliese verwendet werden. Diese PES-Materialien weisen, wie auch das PES- und rPES-Material, ein(e) hervorragende(s) biologische(s) Resorptionsvermögen und/oder Bioaktivität auf. Auch eignen sich diese PES-Materialien bestens für die dauerhafte Lagerung, den Transport und Vertrieb.

Vor Verwendung der PES-Materialien, vorzugsweise unmittelbar vor ihrer Verwendung, z.B. als biologisch resorbierbare und/oder bioaktive Materialien in der Humanmedizin oder Medizintechnik (z.B. für die Wundbehandlung, Wundheilung, als chirurgisches Nahtmaterial oder als Verstärkungsfasern; siehe auch nächster Absatz unten) werden die PES-Materialien (Faser, Pulver, Monolith, Beschichtungslösung) vorzugsweise gewässert, besonders bevorzugt unter leichtem äußeren Druck gewässert. Durch das Wässern werden die restlichen noch vorhandenen Ethoxy-Gruppen vollständig hydrolysiert, die Materialien damit hydrophiler gemacht. Wie oben bereits erwähnt, kann diese Wässerung unter pH-erhöhenden Bedingungen (z.B. in einem Phosphatpuffer H₂PO₄⁻/HPO₄²⁻) erfolgen, insbesondere wenn die Anhebung des pH-Wertes nicht bereits in einem vorherigen Schritt erfolgte. Dabei läuft die fünfte und letzte HKR ab, während der die noch verbliebenen nicht-hydrolysierten Ethoxy-Gruppen aus den PES-Materialien entfernt werden.

Ein weiterer Vorteil ist, dass das erfindungsgemäß erzeugte PES- bzw. rPES-Material und die daraus bestehenden Materialien gegenüber den Fasern und Fasermaterialien, die nach dem Verfahren der DE 196 09 551 C1 erhalten wurden, in Cytotoxizitätstests deutlich verbesserte Werte aufweisen. Diese Verbesserung wurde in Tests in Gegenwart von L929-Mausfibroplasten nachgewiesen. Die Materialien, die erfindungsgemäß aus den Schritten (e1) bis (e4) erhalten werden, zeichnen sich daher durch eine besonders gute biologische Verträglichkeit aus.

Die erfindungsgemäß hergestellten Fasern oder Vliese können insoweit vorteilhaft als biologisch resorbierbare und/oder bioaktive Materialien in der Humanmedizin, der Medizintechnik, der Filtertechnik, der Biotechnologie oder der Dämmstoffindustrie eingesetzt werden. Insbesondere können die erfindungsgemäß hergestellten Materialien vorteilhaft im Bereich der Wundbehandlung und Wundheilung verwendet werden. Fasern können beispielsweise als chirurgisches Nahtmaterial oder als Verstärkungsfasern eingesetzt werden. Vliese können besonders vorteilhaft bei der Versorgung von oberflächlichen Wunden, bei der Filtration von Körperflüssigkeiten (z.B. Blut) oder im Bereich der Bioreaktoren als Anzuchthilfe verwendet werden.

Die erfindungsgemäßen PES-Materialien aus (e1), (e2), (e3) und (e4), die mit einer biologisch aktiven Substanz beladen sein können, das heißt, neben dem bioaktiven Si-Polymer einen weiteren Wirkstoff enthalten, können diesen an den eigentlichen Wirkort transportieren bzw. die Freisetzung des Wirkstoffs am Wirkort beeinflussen. Diese Materialien werden im Folgenden als Wirkstoffträger (drug delivery system) bezeichnet.

Die Verwendung des erfindungsgemäßen gereiften PES-Materials und der erfindungsgemäßen PES-Materialien hat den Vorteil, dass beide vielseitig verarbeitet, eingesetzt und mit verschiedenen (weiteren) Wirkstoffen kombiniert werden können. Besonders bevorzugt ist es, wenn das erfindungsgemäße rPES-Material hierbei keine Reaktionsprodukte mit dem (weiteren) Wirkstoff bildet. Die erfindungsgemäßen PES-Materialien sind biologisch resorbierbar und/oder bioaktiv und weisen verbesserte Cytotoxizitätswerte auf, was zu einer verbesserten biologischen Verträglichkeit der Materialien beiträgt, die gerade im Bereich der Medizin und Medizintechnik notwendig ist.

Die Erfindung soll mit folgendem Beispiel näher erläutert werden, ohne hierauf beschränkt zu sein.

Alle angegebenen Viskositäten wurden mit einem Viskosimeter MCR 300 der Firma Physika bei einer Scherrate von 10 s⁻¹ bei 4°C gemessen.

### Beispiel 1: Biologisch resorbierbares und/oder bioaktives rPES-Material (Sol) und dessen Verarbeitung zu Fasern und Vliesen

Als Edukt für die Hydrolyse-Kondensationsreaktion wurden 2,7 Mol TEOS (Tetraethoxysilan) (562,4 g) in einem Reaktionsgefäß vorgelegt. 3,4 (2,7 x 1,26) Mol EtOH (156,8 g) als Lösungsmittel wurden zugegeben. Das Gemisch wird gerührt. Separat wurde 1 N HNO₃ (27,81 g) mit H₂O (60,38 g) verdünnt. Von dieser verdünnten Salpetersäure wurden anschließend 89.28 g zu dem kompletten TEOS-EtOH-Gemisch bei RT gegeben, so dass das resultierende Reaktionsgemisch 1,8 Mol H₂O und 0,01 Mol HNO₃ pro Mol TEOS enthält. Das Gemisch wurde 5 Stunden gerührt.

Das nach Schritt (a) erhaltene Gemisch wurde nachfolgend durch Eindampfen im Rotationsverdampfer (Schritt b) bei 70°C unter Anlegen eines Vakuums von 500 mbar unter langsamem Rühren (20 U/min) nahezu Wasser- und Ethanol-frei gemacht. Durch die hohe Temperatur wurde die HNO₃ in der reduzierten Form NO₂ stark reduziert. Das Sol wies eine Viskosität von etwa 1 Pa·s (Scherrate von 10 s⁻¹ bei 4°C) auf, die Säure-Stärke nahm stark ab.

Die Lösung wurde in Schritt (c) in einem geschlossenen Polypropylen-Becher (Reifebecher) innerhalb von 30 Minuten auf 4°C abgekühlt und bei dieser Temperatur in Schritt (d) im Reifebecher einer Reifung von 8 Tagen unterworfen. Es wurde eine homogene einphasige Sol-Masse mit einer Viskosität von ca. 40 Pa·s (Scherrate 10 s⁻¹ bei 4°C) erhalten. Das Sol lag ohne erkennbaren festen Phasenanteil vor.

Das Sol konnte im Schritt (e1) zu Fasern versponnen werden. Es wird auch als Spinnmasse bzw. rPES-Material bezeichnet. Die Herstellung der Fasern erfolgte in einer üblichen Spinnanlage. Dazu wurde die Spinnmasse in einen auf-15°C gekühlten Druckzylinder gefüllt, der mit einem Druck von 20 bar beaufschlagt wurde. Die daraus resultierende Kraft presste die Spinnmasse durch Düsen. Die austretende Spinnmasse (Strahl) wies je nach Düsendurchmesser einen Durchmesser von 50 bis 100 µm auf. Der zerfließliche, honigartige Strahl fiel durch sein Eigengewicht in einen unter dem Druckzylinder befindlichen Spinnschacht mit 2 m Länge und reagierte dort mit der Luftfeuchtigkeit zu einer formstabilen, im Querschnitt runden (nicht ovalen oder gar knochenförmigen) Faser ohne Wellenprofil. Im Spinnschacht wurden Temperatur und Feuchtigkeit kontrolliert eingestellt. Die Temperatur lag bei 20°C und die Luftfeuchte bei 35 %. Es wurden formstabile Fasern gebildet. Die Fasern waren an ihrer Oberfläche noch leicht reaktiv. Die postulierte Zusammensetzung der Fasern ist [OSi₈O₁₂(OH)₂(EtO)₅] (Oligosilsesquioxan). Beim Auftreffen auf den Changiertisch verklebten die Fasern an den Berührungsflächen miteinander zu Fasergelegen (Vliesen). Die Vliese wurden anschließend in einem Trockenschrank bei ca. 30°C gelüftet und das eingeschlossene NO₂ weiter reduziert. Die Säure-Stärke wurde dabei auf ein physiologisch verträgliches Maß reduziert.

Das in Beispiel 1 hergestellte Vlies wurde einem Cytotoxizitäts-Test nach ISO 10993-5 (1999); EN 30993-5 (1994) unterzogen. Die gemessene Cytotoxizität im Vergleich zu den für die Kontrollen ermittelten Werten ergab, dass das erfindungsgemäß hergestellte Vlies keine cytotoxischen Eigenschaften aufwies.

### Vergleichsbeispiel

Die Edukte TEOS (Tetraethoxysilan), EtOH, H₂O und HNO₃ wurden im molaren Verhältnis von 1:1,26 : X : 0.01 (mit X = 1,6, 1,7, 1,8, 1,9 und 2,0) vermischt und 5 Stunden bei Raumtemperatur kräftig gerührt Die resultierenden Lösungen wurden in offenen Gefäßen in ein auf 70°C temperiertes Wasserbad gehängt, worin sie bis zu einem definierten Gewichtsverlust verblieben. Anschließend wurde gekühlt und über ein Edelstahlnetz mit einer Maschenweite von 1 mm x 1mm filtriert. Das Filtrat wurde in einem verschlossenen Gefäß bei einer Temperatur von 3°C einer Reifezeit von 6 Stunden bis 6 Monaten, in Abhängigkeit von Gewichtsverlust, ausgesetzt.

Die resultierende Spinnmasse war sehr homogen und einige Zeit stabil und spinnbar. Die Herstellung der Fasern erfolgte an einer Trockenspinnanlage. Dazu wurde die Spinnmasse in einen auf -15°C gekühlten Spinnkopf gefüllt, bei einem Druck von 10 bis 15 bar zunächst durch ein Edelstahlnetz mit einer Maschenweite von 80 x 80 µm und dann durch eine Düse mit einem Durchmesser von 100 µm gepresst. Der resultierende Endlosfaden wurde nach einer Trockenstrecke von 1 m auf einen rotierenden Zylinder gewickelt. Die resultierenden Fasern zeigen in Abhängigkeit vom Ansatz, d. h der zugegebenen Wassermenge, runde, ovale oder knochenförmige Querschnittformen mit Durchmessern zwischen 5 µm und 30 µm. Die Querschnittsflächen liegen zwischen 100 µm² bis 400 µm².

Die Faseroberfläche ist glatt und zeigt in keinem Fall ein Wellenprofil. Zugfestigkeitsmessungen der Fasern ergaben Werte von 100 MPa bis 800 MPa. Vom Fasermaterial angefertigte IR-Spektren zeigen eine Si-OH-Bande bei 950 cm⁻¹ und C-H-Signale bei 3000 cm⁻¹. Es liegt also eine teilhydrolysierte und teilkondensierte Ethoxy-Silanol-Fasem vor, Nach ca. 2 Monaten Lagerung bei Raumtemperatur sind im IR-Spektrum keine C- H-Schwingungsbanden mehr zu erkennen. Die Fasern haben sich in teilkondensierte Silanol-Fasern umgewandelt, welche über einen Zeitraum von mehreren Monaten stabil sind.

Mit den so hergestellten Fasern wurden Cytotoxizitätsmessungen durchgeführt. Bei dem hieraus erzeugten Fasermaterial wurden im Cytotoxizitätstest nach ISO 10993-5 (1999); EN 30993-5 (1994) cytotoxische Wirkungen festgestellt.

Auch konnte hierbei nur 50 % des gesamten Reaktionsansatzes versponnen werden.

## Patentansprüche

1. Polyethoxysiloxan (PES)-Material, dadurch zu erhalten, dass
(a) eine erste Hydrolyse-Kondensationsreaktion (HKR) von höchstens *einem* Rest X von einer oder von mehreren verschiedenen Si-Verbindungen der Formel I
SiX₄ (I),
in der die Reste X gleich oder verschieden sind und Hydroxy, Wasserstoff oder Ethoxy (EtO) bedeuten, sauer katalysiert bei einem anfänglichen pH-Wert von 0 bis ≤ 7, in Gegenwart von Ethanol (EtOH) oder eines Ethanol-Wasser-Gemisches als Lösungsmittel, über einen Zeitraum von 1 bis 24 h bei einer Temperatur von 0°C bis 78°C (Siedepunkt des Ethanol) durchgeführt wird,
(b) eine zweite HKR des in Schritt (a) erhaltenen Materials bei gleichzeitigem Entfernen des Lösungsmittels durch sukzessives Eindampfen in einem gasdiffusionsdichten Behälter bei einem Druck von 100 bis 1013 mbar, vorzugsweise bei einem leichten Unterdruck von 300 mbar bis 800 mbar, einer Temperatur von 50-78°C, bevorzugt von etwa 70°C, bis zu einer drastischen Viskositätserhöhung auf 0,5 bis 2, vorzugsweise 1 Pa·s bei einer Scherrate von 10 s⁻¹ bei 4°C bis zur Gewichtskonstanz und bis zur Bildung eines Cyclotetrasiloxans der allgemeinen Formel ((SiO(OH)_{0,75}(OEt)_{1,25} x 1/64 H₂O)₄ und der Molmasse von 4 * ca. 114 g = ca. 456 g, durchgeführt wird;
(c) dieses PES-Material in einem geschlossenen, vorzugsweise gasdiffusionsdichten Behälter in einem Zeitraum von 2 bis 5 Minuten bis 0,2 bis 5, vorzugsweise 0,5. Stunden abgekühlt wird, und
(d) das aus (c) erhaltene PES-Material durch eine dritte HKR in ein rPES (gereiftes PES), Material überführt wird.

2. Material nach Anspruch 1, **dadurch gekennzeichnet, dass** der pH von 0 bis ≤ 7 in Schritt (a) mit verdünnter Salpetersäure oder mit einem sauren Gemisch oder Lösung aus (i) einer physiologisch verträglichen Säure wie Zitronen-, Bernstein-, Wein-, Essig- oder Ascorbinsäure und (ii) einem Substrat der Nitroxid-Synthase (NOS) wie Arginin eingestellt wird.

3. Material nach Anspruch 2, **dadurch gekennzeichnet, dass** die verdünnte Salpetersäure in einem molaren Verhältnis Si-Verbindung(en) der Formel (I) zu Salpetersäure von 90:1 bis 110: 1, bevorzugt von 100:1 eingesetzt wird.

4. Material nach Anspruch 1, **dadurch gekennzeichnet, dass** in Schritt (b) die Säure-Stärke verringert wird, was insbesondere durch das Eindampfen oder das Abdampfen von NO₂ oder mittels einer Tris-Lösung erfolgt.

5. Material nach einem der vorhergehenden Ansprüche, **dadurch gekennzeichnet, dass** das Sol in Schritt (c) auf -20°C bis +10°C, bevorzugterweise auf +2°C bis +4°C oder auf -20°C bis -10°C, besonders bevorzugt auf+4°C abgekühlt wird.

6. Material nach einem der vorhergehenden Ansprüche, **dadurch gekennzeichnet, dass** die Reifung in Schritt (d) bei einer Temperatur von -20°C bis 10°C, bevorzugt von 2°C bis 4°C, besonders bevorzugt bei 4°C erfolgt.

7. Material nach einem der Ansprüche 1 bis 6, **dadurch gekennzeichnet, dass** Schritt (d) bis zu einer Viskosität (bei einer Scherrate von 10 s⁻¹ bei 4°C) des Materials von 30 bis 55 Pa·s, vorzugsweise von etwa 40 Pa·s, durchgeführt wird.

8. Material nach einem der vorhergehenden Ansprüche, **dadurch gekennzeichnet, dass** das gereifte PES-Material in einem Schritt (e1) zu biologisch resorbierbaren und/oder bioaktiven Fasern versponnen wird.

9. Material nach einem der Ansprüche 1 bis 7, **dadurch gekennzeichnet, dass** das gereifte PES-Material in einem weiteren Schritt (e2), (e3) oder (e4) zu einem/r biologisch resorbierbaren und/oder bioaktiven Pulver; Monolith oder Beschichtung verarbeitet wird.

10. Material nach Anspruch 8 oder 9, **dadurch gekennzeichnet, dass** in den Schritten (e1), (e2), (e3) bzw. (e4) die Säure-Stärke verringert wird, was insbesondere durch das Eindampfen oder das Abdampfen von NO₂ oder mittels einer Tris-Lösung erfolgt.

11. Material nach einem der vorhergehenden Ansprüche, **dadurch gekennzeichnet, dass** die in Schritt (a) eingesetzte Si-Verbindung Tetraethoxysilan (TEOS) ist.

12. Verwendung des Materials nach einem der Ansprüche 1 bis 11 für die Herstellung biologisch resorbierbarer und/oder bioaktiver PES-Materialien.

13. Verwendung nach Anspruch 12, wobei die PES-Materialien Fasern, Vliese, Pulver, Monolithe oder Beschichtungslösungen sind.

14. Verwendung nach Anspruch 13, wobei die Fasern, Vliese, Pulver, Monolithe oder Beschichtungslösungen unmittelbar vor ihrer Verwendung gewässert werden.

## Claims

1. Polyethoxysiloxane (PES) material obtainable by
(a) performing a first hydrolysis-condensation reaction (HCR) of at most one X radical of one or more different silicon compounds of formula I
SiX₄ (I),
where the X radicals are the same or different and each represent hydroxyl, hydrogen or ethoxy (EtO), acid catalyzed at an initial pH of 0 to ≤ 7, in the presence of ethanol (EtOH) or of an ethanol-water mixture as solvent, for a period of 1 to 24 h at a temperature of 0°C to 78°C (boiling point of ethanol),
(b) performing a second HCR of the material obtained in step (a) while at the same time removing the solvent by gradual evaporation in a gas diffusion tight container at a pressure of 100 to 1013 mbar, preferably at a slight underpressure of 300 mbar to 800 mbar, a temperature of 50-78°C, preferably about 70°C, until a drastic increase in viscosity to 0.5-2, preferably 1, Pa·s at a shear rate of 10 s⁻¹ at 4°C, until weight constancy and until formation of a cyclotetrasiloxane of the general formula ((SiO(OH)_{0,75}(OEt)₁.₂₅ × 1/64 H₂O)₄ and of molar mass 4 * about 114 g = about 456 g;
(c) cooling this PES material in a closed, preferably gas diffusion tight container over a period of 2 to 5 minutes to 0.2 to 5, preferably 0.5 hours, and
(d) converting the PES material obtained from (c) into an rPES (ripened PES) material via a third HCR.

2. Material according to Claim 1, **characterized in that** the pH of 0 to ≤ 7 in step (a) is set with dilute nitric acid or with an acidic mixture or solution of (i) a physiologically tolerable acid such as citric, succinic, tartaric, acetic or ascorbic acid and (ii) a substrate of nitroxide synthase (NOS) such as arginine.

3. Material according to Claim 2, **characterized in that** the dilute nitric acid is used in a molar ratio of silicon compound(s) of formula (I) to nitric acid in the range from 90:1 to 110:1, preferably 100:1.

4. Material according to Claim 1, **characterized in that** the acid strength is reduced in step (b), particularly by evaporating NO₂ or by means of a Tris solution.

5. Material according to any preceding claim, **characterized in that** the sol in step (c) is cooled down to -20°C to +10°C, preferably to +2°C to +4°C or to -20°C to -10°C, more preferably to +4°C.

6. Material according to any preceding claim, **characterized in that** the ripening in step (d) is effected at a temperature of -20°C to 10°C, preferably of 2°C to 4°C, more preferably at 4°C.

7. Material according to any one of Claims 1 to 6, **characterized in that** step (d) is performed until a viscosity (at a shear rate of 10 s⁻¹ at 4°C) of the material in the range from 30 to 55 Pa·s, preferably of about 40 Pa·s.

8. Material according to any preceding claim, **characterized in that** the ripened PES material is spun in a step (e1) into bioabsorbable and/or bioactive fibers.

9. Material according to any one of Claims 1 to 7, **characterized in that** the ripened PES material is processed in a further step (e2), (e3) or (e4) into a bioabsorbable and/or bioactive powder, monolith or coating.

10. Material according to Claim 8 or 9, **characterized in that** the acid strength is reduced in the steps (e1), (e2), (e3) and/or (e4), particularly by evaporating NO₂ or by means of a Tris solution.

11. Material according to any preceding claim, **characterized in that** the silicon compound used in step (a) is tetraethoxysilane (TEOS).

12. Use of the material according to any one of Claims 1 to 11 for producing bioabsorbable and/or bioactive PES materials.

13. Use according to Claim 12, wherein the PES materials are fibers, fibrous nonwoven webs, powders, monoliths or coating solutions.

14. Use according to Claim 13, wherein the fibers, fibrous nonwoven webs, powders, monoliths or coating solutions are watered immediately before use.

## Revendications

1. Matériau à base de polyéthoxysiloxane (PES), obtenu en ce que
(a) une première réaction d'hydrolyse-condensation (HKR) d'au plus un radical X d'un ou de plusieurs composés de Si différents de formule 1
SiX₄ (I),
dans laquelle les radicaux X sont identiques ou différents et signifient hydroxy, hydrogène ou éthoxy (EtO), est réalisée sous catalyse acide à un pH initial de 0 à ≤ 7, en présence d'éthanol (EtOH) ou d'un mélange éthanol-eau en tant que solvant, pendant une durée de 1 à 24 h à une température de 0 °C à 78 °C (point d'ébullition de l'éthanol),
(b) une deuxième HKR du matériau obtenu à l'étape (a) est réalisée avec élimination simultanée du solvant par concentration successive dans un contenant étanche à la diffusion des gaz à une pression de 100 à 1 013 bar, de préférence à une légère sous-pression de 300 mbar à 800 mbar, à une température de 50 à 78 °C, de préférence d'environ 70 °C, jusqu'à une augmentation de viscosité importante à 0,5 à 2, de préférence 1 Pa·s, à un taux de cisaillement de 10 s⁻¹ à 4 °C, jusqu'au poids constant et jusqu'à la formation d'un cyclotétrasiloxane de formule générale ((SiO(OH)_{0,75}(OEt)_{1,25} x 1/64 H₂O)₄ et de masse molaire de 4 * environ 114 g = environ 456 g ;
(c) ce matériau à base de PES est refroidi dans un contenant fermé, de préférence étanche à la diffusion des gaz, pendant une durée de 2 à 5 minutes à 0,2 à 5, de préférence 0,5, heures, et
(d) le matériau à base de PES obtenu en (c) est transformé en un matériau à base de rPES (PES mûri) par une troisième HKR.

2. Matériau selon la revendication 1, **caractérisé en ce que** le pH est ajusté de 0 à ≤ 7 à l'étape (a) avec de l'acide nitrique dilué ou avec un mélange ou une solution acide de (i) un acide physiologiquement compatible tel que l'acide citrique, l'acide succinique, l'acide tartrique, l'acide acétique ou l'acide ascorbique, et (ii) un substrat de nitroxyde synthase (NOS) tel que l'arginine.

3. Matériau selon la revendication 2, **caractérisé en ce que** l'acide nitrique dilué est utilisé en un rapport molaire entre le ou les composés de Si de formule (I) et l'acide nitrique de 90:1 à 110:1, de préférence de 100:1.

4. Matériau selon la revendication 1, **caractérisé en ce que** la force de l'acide est réduite à l'étape (b), ce qui a lieu notamment par la concentration ou l'évaporation de NO₂ ou par une solution Tris.

5. Matériau selon l'une quelconque des revendications précédentes, **caractérisé en ce que** le sol à l'étape (c) est refroidi à une température de -20 °C à +10 °C, de préférence de +2 °C à +4 °C ou de -20 °C à -10 °C, de manière particulièrement préférée à +4 °C.

6. Matériau selon l'une quelconque des revendications précédentes, **caractérisé en ce que** la maturation à l'étape (d) a lieu à une température de -20 °C à 10 °C, de préférence de 2 °C à 4 °C, de manière particulièrement préférée à 4 °C.

7. Matériau selon l'une quelconque des revendications 1 à 6, **caractérisé en ce que** l'étape (d) est réalisée jusqu'à une viscosité (à un taux de cisaillement de 10 s⁻¹ à 4 °C) du matériau de 30 à 55 Pa-s, de préférence d'environ 40 Pa·s.

8. Matériau selon l'une quelconque des revendications précédentes, **caractérisé en ce que** le matériau à base de PES mûri est filé lors d'une étape (e1) en des fibres biologiquement résorbables et/ou bioactives.

9. Matériau selon l'une quelconque des revendications 1 à 7, **caractérisé en ce que** le matériau à base de PES mûri est transformé lors d'une étape supplémentaire (e2), (e3) ou (e4) en une poudre biologiquement résorbable et/ou bioactive ; un monolithe ou un revêtement.

10. Matériau selon la revendication 8 ou 9, **caractérisé en ce que** la force de l'acide est réduite lors des étapes (e1), (e2), (e3) et (e4), ce qui a lieu notamment par la concentration ou l'évaporation de NO₂ ou par une solution Tris.

11. Matériau selon l'une quelconque des revendications précédentes, **caractérisé en ce que** le composé de Si utilisé à l'étape (a) est le tétraéthoxysilane (TEOS).

12. Utilisation du matériau selon l'une quelconque des revendications 1 à 11 pour la fabrication de matériaux à base de PES biologiquement résorbables et/ou bioactifs.

13. Utilisation selon la revendication 12, dans laquelle les matériaux à base de PES sont des fibres, des non-tissés, des poudres, des monolithes ou des solutions de revêtement.

14. Utilisation selon la revendication 13, dans laquelle les fibres, non-tissés, poudres, monolithes ou solutions de revêtement sont hydratés directement avant leur utilisation.
